# EUROPEAN PATENT SPECIFICATION

(11) **EP 2 394 950 B1**
(45) Date of publication and mention of the grant of the patent: **05.04.2017**
(21) Application number: 10738578.3
(22) Date of filing: 04.02.2010
(51) Int. Cl.: A61L 2/07, A61L 2/24, B65B 55/02, A61L 2/20, A61L 2/22, B29C 49/42, B65B 55/10, B67C 7/00

(54) **BEVERAGE FILLING METHOD AND DEVICE**
GETRÄNKEAUSGABEVERFAHREN UND -VORRICHTUNG
PROCÉDÉ ET DISPOSITIF DE REMPLISSAGE DE BOISSON

(30) Priority: 06.02.2009 JP 2009026034; 10.03.2009 JP 2009056701
(43) Date of publication of application: 14.12.2011
(60) Divisional application: 17156642.5
(73) Proprietor: Dai Nippon Printing Co., Ltd., Tokyo 162-8001 (JP)
(72) Inventor: HAYAKAWA Atsushi, Tokyo 162-8001 (JP); NAKAMURA Yuiko, Tokyo 162-8001 (JP)
(74) Representative: Viering, Jentschura & Partner mbB Patent- und Rechtsanwälte
(86) International application number: PCT/JP2010/051579
(87) International publication number: WO 2010/090247

(56) References cited:
- EP-A2- 2 138 298
- DE-A1-102008 057 403
- JP-A- 8 282 789
- JP-A- 11 198 916
- JP-A- H08 282 789
- JP-A- 2000 326 935
- JP-A- 2001 212 874
- JP-B- 6 015 202
- JP-B2- 3 801 692
- JP-T- 2002 502 669
- JP-T- 2003 514 624
- JP-T- 2008 543 619

## Description

### [Field of the Invention]

The present invention relates to a beverage filling method and a beverage filling system.

### [Background Art]

As a conventional technology, there is provided aseptic filling methods, as an inline system for manufacturing an aseptic package, comprising the steps of sterilizing a preform made of such as PET (polyethylene terephthalate) during a conveyance thereof, molding the preform into a bottle by a blow molding machine, filling beverage into the bottle and capping the bottle, such method being disclosed, for example, in Japanese Patent Documents 1, 2, 3, 5, 6, 7 and 8. These methods aim to perform a sterilizing treatments or processing in a preform forming stage before molding a bottle in place of a sterilizing treatment or processing performed after the bottle molding process.

Japanese Patent Documents 1, 5, 6 and 7 provide a technology in which a sterilizing agent, in a gaseous state, such as hydrogen peroxide is blown to a preform before blow-molding process to thereby sterilize the preform, then the preform is heated to a temperature suitable for draw-stretching, and thereafter, the stretching blow molding is performed. The Japanese Patent Document 2 provides a method in which an evaporated sterilizing agent is adhered to the preform and remaining sterilizing agent is discharged by flame of a burner, and the Japanese Patent Document 3 provides a method in which hydrogen peroxide steam is sprayed onto the preform, which is then heated and subjected to a blow-molding process. The Japanese Patent Document 8 provides a method in which the preform is dipped into the hydrogen peroxide solution to thereby sterilize the same.

Furthermore, as a method of sterilizing the preform, there is also provided a method, for example, as disclosed in the Japanese Patent Document 4, in which water steam is blown into a preform instead of a sterilizing agent, the preform is sterilized by maintaining a temperature more than glass transition temperature for a predetermined time interval, and immediately thereafter, gas such as air or nitrogen gas is blown into the preform to remove water or water moisture inside the preform.

Still furthermore, there is also provided a method in which a bottle is washed and cleaned by a contamination removing agent at an outlet portion of a blow-molding machine in order to prevent contaminated fine particles from entering at the blow-molding process as disclosed, for example, in the Japanese Patent Document 9, paragraph 0076.

### Prior Art Documents

### Patent Documents

Patent Document 1: Japanese Patent Application Laid-open Publication No. 2001-212874
Patent Document 2: Japanese Patent Publication No. 3780165
Patent Document 3: Japanese Patent Application Laid-open Publication No. 2008-183899
Patent Document 4: Japanese Patent Application Laid-open Publication No. 2007-111886
Patent Document 5: Japanese Patent Application Laid-open Publication No. 2008-543619
Patent Document 6: Japanese Patent Application Laid-open Publication No. 2008-546605
Patent Document 7: Japanese Patent Application Laid-open Publication No. HEI 8-2885869
Patent Document 8: Japanese Patent Publication No. 2885869
Patent Document 9: Japanese Patent Publication No. 3903411

A beverage filling method and system with a two-stage sterilization of preform and container is disclosed in Japanese Patent Publication No. 8-282789.

### Disclosure of the Invention

### Problem to be solved by the Invention

However, if the blow-molding process is performed after the blowing of a gas having high density (concentration) such as hydrogen peroxide to thereby sterilize the preform as performed in a conventional technology, respective parts or components of a molding machine have been likely corroded by the sterilizing agent.

Moreover, many of the oxide sterilizing agents includes phosphoric acid series chelate agent for stabilizing density or concentration, If such sterilizing agent is introduced into the molding machine with being adhered to the preform and then gasified, it causes the risk that the chelate agent adheres to and piles on the molding machine, thus contaminating the molding machine and mixing, as foreign material, into a container such as bottle.

Furthermore, after the adhesion of the sterilizing agent to the preform, when the sterilizing agent is heated by flame of a burner to be removed from the preform, such process is complicated and a system or device for performing such process is also complicated, which may result in cost increasing.

Still furthermore, according to the method of sterilizing the interior of the preform by steam without using any sterilizing agent, it is necessary to maintain the preform at a predetermined temperature for a predetermined time interval till the sterilization has been completed, and it is also necessary to perform a water removing process after the sterilization, thus being inconvenient.

Still furthermore, in the conventional technology, there is the risk that bacteria or like is invaded into the bottle at the time of the blow-molding process by high-pressure (highly pressurized) air or conveying the bottle to a filling area after the sterilization by the adhesion of the sterilizing agent to the preform. Although it is not impossible to sterilize all of the high-pressure air circuit in the blow-molding machine, a stretching rod, a die, and a bottle conveying area before manufacturing the bottle and to maintain an aseptic atmosphere, in such case, it is required to additionally install a sterilizing system or like and exchange many of parts with those made of anti-chemical materials, which requires huge amount of initial investment cost.

The method in which a bottle is washed by a contamination removing agent at an outlet of the blow-molding machine may be available, but the sterilization of the interior of the bottle may be likely incompletely performed. In addition, since a drying process of the contamination removing agent is also required, a system including the blow-molding machine may become complicated and large in structure, thus being inconvenient.

In addition, even if a preform to which the sterilizing agent adheres is heated and put on a mandrel or spindle, a mouth portion of the preform is heated to a temperature of mere 40 to 50 °C, and hence, the mouth portion is heated to a temperature less than the other portions of the bottle, and hence, less sterilizing effect is achieved, thus the sterilizing effect being insufficient That is, if the mouth portion of the preform is heated to a high temperature, the mouth portion may be deformed, and accordingly, when a cap is applied, bottle sealing performance may not be maintained, and therefore, it is necessary to set a limit to heating temperature to the mouth portion within the above-mentioned temperature range.

The present invention aims to solve the problems or inconveniences mentioned above.

### Means for solving the Problem

The present invention provides a beverage filling method according to claim 1 and a beverage filling system according to claim 4. Further advantageous embodiments of the present invention are disclosed in the dependent claims.

### Effects of the Invention

In the present invention, the beverage filling method includes the steps of:, while continuously conveying the preform (1), forming a water film on a surface of a preform (1) by spraying water steam (W); heating and drying the preform (1) under a state in which the water film or condensed film of the hydrogen peroxide is adhered to the surface of the preform to preliminarily sterilize the preform (1); producing a container (2) through a blow-molding process within a blow-molding die (4) which is continuously being transferred; taking out the container (2) from the blow-molding die (4), while continuously transferring the container, main sterilization is performed ; and filling, thereafter, the container (2) with a beverage (a) and sealing the container (2) with a lid (3).

Accordingly, by performing the preliminary sterilization as heat humidity sterilization, it is possible to sterilize mold, having heat resisting property, fungus such as yeast, Gram-negative bacteria (salmonella, coli bacteria or like) and the like bacteria. Therefore, when it is required to be filled up with acidic drink, mineral water, carbonated drink and the like, the sterilizing process by the main sterilization can be simplified. That is, when the main sterilization is performed by using a sterilizing agent such as hydrogen peroxide, acetyl peroxide, chlorine water, ozone or like sterilizing agent, the amount thereof to be used, temperature, and density thereof can be reduced. In a case when the hot water is utilized, the amount thereof to be used and the temperature can be also reduced. In a case when a UV lamp is utilized, irradiation dose can be reduced. Moreover, since the using amount of the hydrogen peroxide can be reduced in the main sterilization process, when the preform (1) and the container (2) are formed of PET material capable of easily absorbing the hydrogen peroxide, an excessive adsorption of the hydrogen peroxide to the container (2) can be effectively prevented.

Furthermore, in the present invention, the beverage filling method includes the steps of:, while continuously conveying the preform (1), forming a water film on a surface of a preform (1) by spraying hydrogen peroxide mist or gas (K) to form a condensed film of hydrogen peroxide on the surface of the preform (1); and heating and drying the preform (1) under a state in which the water film or condensed film of the hydrogen peroxide is adhered to the surface of the perform to preliminarily sterilize the preform (1), a general molding die may be used with no problem, and it is possible to sterilize, at portions other than a mouth portion (2a) of the container, general bacterial, sporeforming bacteria, fungus such as mold or yeast and the like.

Furthermore, in the present invention, in the case when the main sterilization process is performed by blowing condensed mist (M) or gas (G) of hydrogen peroxide to the container (2) to which the heat by the heating to the preform (1) remains, and subsequently, an air rinsing process is performed by rinsing aseptic air, the spore-forming bacteria can be sterilized, so that the beverage having low acidic property more than pH 6 can be filled. In addition, since the blow-molding process is performed after the sterilization of the preform by using water steam and the thus blow-molded container (2) is sterilized by the hydrogen peroxide, corrosion, degradation and the like defective of the blow-molding die by the adhesion of the hydrogen peroxide can be prevented from occurring.

Furthermore, in the present invention, in the case when the main sterilization process is performed by the hot water rinsing using the aseptic hot water (H), a problem of the remaining of the hydrogen peroxide to the container (2) can be solved, and the invention becomes suitable for the filling of the beverage such as mineral water or acid-beverage (exception of low acid-beverage ). Moreover, in the case when the preliminarily sterilization is performed by the hydrogen peroxide having extremely low density, the main sterilization will be performed by the hot rinsing process using the hot water (H). In such case, the problem of the remaining of the hydrogen peroxide to the container (2) can be solved, and the invention is suitably applicable for the filling of the beverage such as mineral water or acid-beverage (exception of low acid-beverage).

Furthermore, in the present invention, in the case when a water film is adhered to each of the preforms (1) of an amount within 0.02mg/cm² to 1.15mg/cm² by blowing water steam (W) to the preform, the causing of the defective sterilization to the container (2) can be prevented, and the defect in the molding process such as whitening, distortion uneven molding or the like can also be prevented.

Still furthermore, in the present invention, in the case when a condensed film of the hydrogen peroxide of 35 weight % (reduced quantity) is adhered to each of the preforms of an amount within 0.0035 *µ*L/cm² to 0.35 *µ*L /cm² by blowing the hydrogen peroxide mist or gas (L) to the preform, the causing of the defective sterilization to the container (2) can be prevented, and the defect in the molding process such as whitening, distortion uneven molding or the like can be also prevented.

### Brief Description of the Drawings

[Fig. 1] is a front view, partially cut away, showing one example of a package manufactured by a filling method and a filling system according to the present invention.
[Fig. 2] is a view explaining respective processes in a first half of a filling method according to embodiments 1 and 2 (first and second embodiments) of the present invention.
[Fig. 3] is a view explaining respective processes in a latter half of the filling method according to the embodiment 1 of the present invention.
[Fig. 4] is a vertical view, partially in section, showing one example of a generator for generating hydrogen peroxide mist or gas.
[Fig. 5] is a plan view showing a schematic structure of one example of the filling system according to the first embodiment.
[Fig. 6] is a view explaining respective processes in a latter half of the filling method according to the embodiment 2 of the present invention.
[Fig. 7] is a plan view showing a schematic structure of one example of the filling system according to the second embodiment.
[Fig. 8] is a view explaining processes different from those in the embodiments 1 and 2 in a filling method according to a third embodiment 3 of the present invention.

### Modes for embodying the Invention

Hereunder, modes for embodying the present invention will be explained.

### <Embodiment 1>

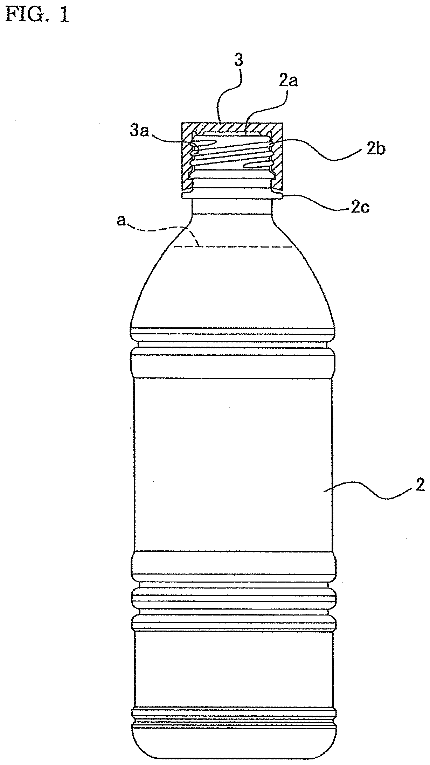

According to an inline system of the first embodiment 1, a package shown in Fig. 1 can be manufactured as a final product.

As shown in Fig. 1, this package is composed of a bottle 2 as a container and a cap 3 as a lid.

In this embodiment, although the bottle 2 is made of PET, another resin such as polypropylene, polyethylene or like may be used for manufacturing the package without limiting to the PET, and a male screw (threaded) portion 2b is formed to a mouth portion 2a of the bottle 2.

The cap 3 is produced by an injection molding process with polypropylene being used as a resin material, and a female screw (threaded) portion 3a is also formed together with the molding of the cap 3.

The bottle 2, the interior of which is preliminarily sterilized, is filled up with beverage (drink) a which had already been sterilized, and after the filling of the beverage a, the cap 3 is applied to the mouth portion 2a of the bottle 2, which is then sealed by the screw-engagement between the male and female screws 2b and 3a, thus completing the package.

The bottle 2 mentioned above is formed in processes, which will be described hereinafter, as a container, and the container is then filled up with the beverage and sealed as the package.

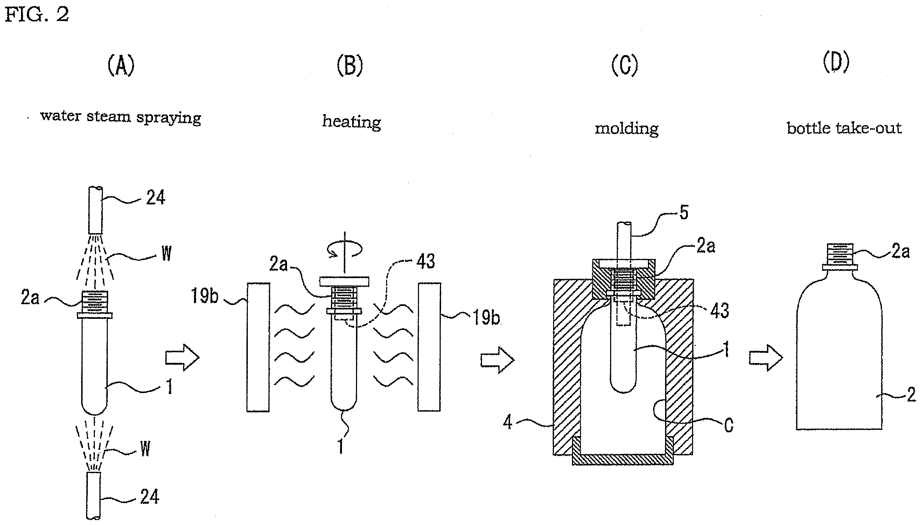

First, the preform 1 shown in Fig. 2(A) is continuously delivered in one direction at a constant speed.

The preform 1 is formed as a bottomed cylindrical body having substantially test tube shape through the PET injection molding process. The preform 1 is formed with a mouth portion 2a like that of the bottle shown in Fig. 1 at a time of initial molding process. This mouth portion 2a is formed with the male screw 2b at the same time of molding the preform 1.

Just after the starting of the conveyance or delivery of the preform 1, water steam W in form of mist is sprayed to the preform 1 from a nozzle 24 as shown in Fig. 2(A) to thereby form a thin water film on the surface of the preform 1.

By spraying the water steam W to the preforms 1, the water films, of an amount of 0.02mg/cm² to 1.15mg/cm², are stuck and formed on the surfaces of the respective preforms 1. In the case of the sticking amount being less than 0.02mg/cm², only dry-heat sterilization is performed, and hence, sufficient sterilizing effect will not be achieved even by performing post heating shown in Fig. 2(B). On the other hand, in the case of the sticking amount being more than 1.15mg/cm², at a time when the blow-molding is thereafter performed as shown in Fig. 2(C), whiting phenomenon, spot, wrinkle , deformation or the like defective phenomenon may be caused to the bottle.

The sticking amount of the water to the preform 1 is preferably 0.07mg/cm² to 0.46mg/cm².

A heater 19b is arranged in form of a tunnel, as shown in Fig. 2(B), along the conveying path of the preforms 1, and the preforms 1 are heated by the heater 19b during the conveyance thereof. The preforms 1 are heated by such heating uniformly to a temperature in a range of 90°C to 120°C suitable for being subjected to the blow molding process.

However, because the mouth portion 2a affects the sealing performance between the mouth portion 2a and the cap 3, the heating temperature for the mouth portion 2a is limited within a range of about 40°C to 50°C for preventing the mouth portion 2a from being deformed by the heating.

When the preform 1 is heated, the preform 1 takes a vertically suspended state with the mouth portion 2a being directed upward (or in an inverted state) by the insertion of a spindle (or mandrel) into the preform 1 and then the preform is conveyed or delivered while being rotated together with the spindle (or mandrel), thereby being uniformly heated by the heater 19b.

Further, during this heating process, the surface of the preform 1 is subjected to a preliminary sterilization. That is, by drying the preform 1 while heating the same in a state of the water film being stuck on the surface of the preform 1, the dry-heat sterilization treatment changes to the wet-heating sterilization treatment, whereby even fungus (mold), which has heat-resisting property and cannot be sterilized at all by the dry-heating sterilization treatment, can be easily sterilized. Thereby, fungus such as heat-resisting fungus or yeast, salmonella fungus, Gram-negative fungus such as bacteria coliform group or like adhering to the surface of the preform 1 can be well sterilized.

The preform 1 heated to a temperature suitable for the blow-molding and then preliminarily sterilized is molded into a bottle 2 as a container subjected to the blow-molding process as shown in Fig. 2(C).

A die 4 as a molding frame for the blow-molding process is continuously transferred at the same running speed as that of the preform 1, the die is subjected to a die clamping respectively closing process, and the blow-molding is performed to the preform 1 in the die 4. Thereafter, the die 4 is opened.

The preform 1 is heated substantially uniformly during the heating process shown in Fig. 2(B) so that the entire temperature of the preform increases to a temperature range suitable for the molding process, and while maintaining such heating (heated) state, the preform 1 together with the spindle 43 is installed in the die 4 as shown in Fig. 2(C). Then, a blowing nozzle 5 is inserted into the preform 1 by penetrating the upper portion of the die frame 4 and the spindle 43 of the mouth portion 2a of the preform 1.

During the conveyance of the die 4, the preform 1 is expanded in a cavity C in the die 4 to a bottle 2 as a final product, for example, by subsequently blowing primary blow air and secondary blow air from the blowing nozzle 5 into the preform 1.

When the bottle 1 is molded in the die 4 by the manner mentioned above, the die 4 is opened while being conveyed, and as shown in Fig. 2(D), the completed product of a bottle is taken outside the die 4.

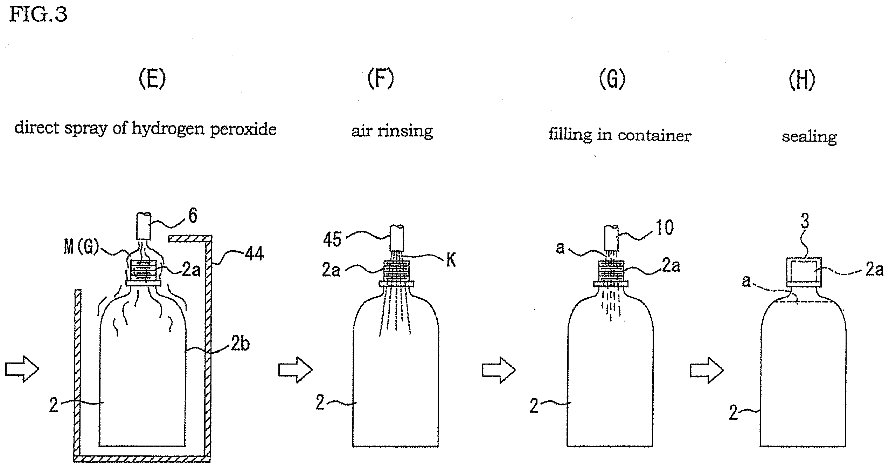

The bottle 2 is continuously conveyed after the molding and is finally sterilized during the conveyance as shown in Fig. 3(E). This main (final) sterilization is performed by blowing mist M or gas G of hydrogen peroxide as the sterilizing agent through the sterilization nozzle 6, which is disposed in opposition to the opening of the mouth portion 2a of the preform 1. The mist M or gas G of the hydrogen peroxide blown out from the sterilization nozzle 6 invades into the bottle 2 through the mouth portion 2a thereof to thereby sterilize the inner surface of the bottle 2.

Further, a tunnel 44 is provided at a portion on a continuously transferring line of the bottle 2, so that the mist M or gas G of the hydrogen peroxide blown out from the sterilization nozzle 6 stays in the tunnel 44 so as to effectively sterilize the outer surface of the bottle 2.

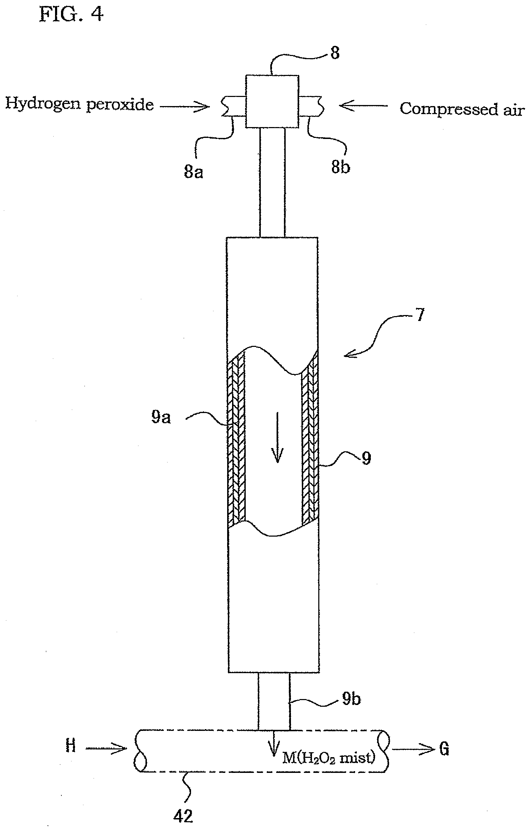

The mist M or gas G of the hydrogen peroxide may be generated by, for example, a generator 7 shown in Fig. 4.

The generator 7 is equipped with a hydrogen peroxide supply unit 8 formed as a two-fluid spray for spraying, in liquid drop state, the solution of the hydrogen peroxide as the sterilizing agent and also equipped with a vaporizer or evaporator 9 for heating sprayed mist of the hydrogen peroxide supplied from the hydrogen peroxide supply unit 8 to a temperature more than boiling point and less than graded point and then vaporizing the same.

The hydrogen peroxide supply unit 8 serves to introduce the hydrogen peroxide solution from a hydrogen peroxide supply passage 8a and compressed air from compressed air supply passage 8b so as to spray the mist of the hydrogen peroxide solution into the mist of the vaporizer 9. The evaporator 9 is composed of a pipe in which a heater 9a is sandwiched between inner and outer wall sections, and the sprayed mist of the hydrogen peroxide blown into the pipe is heated and vaporized. The vaporized hydrogen peroxide gas G is discharged outside the vaporizer 9 through a discharge nozzle 9b as condensed mist M.

The mist M shown in Fig. 3(E) is the condensed mist M. In a case when the gas G is used in place of this mist M, a conduit 42 through which hot air H flows is connected to a tip end portion of the discharge nozzle 9b as shown in Fig. 4 with two-dot-chain line, to gasify the condensed mist M from the discharge nozzle 9b by the hot air H, and this gas G is flown to the sterilization nozzle 6 through a flexible hose or like.

The sterilization nozzle 6 may be located at a constant position on the conveyance path of the bottle 2 or may be moved in synchronous with the bottle 2.

As shown in Fig. 3(E), although the mist M or gas G of the hydrogen peroxide sprayed out of the sterilization nozzle 6 contacts inner and outer surfaces of the bottle 2, the heat applied to the preform 1 remains to the bottle 2 at that time and is maintained to a predetermined temperature, thus being effectively sterilized. This predetermined temperature is preferably 40 to 75 °C in a case where the preform 1 is formed of PET material, and more preferably, 50 to 75 °C. In a case of less than 40 °C, the sterilizing performance is extremely degraded and in a case of more than 75 °C, the molding machine may be damaged.

After the blowing of the mist M or gas G of hydrogen peroxide, the bottle 2 is continuously conveyed or delivered, and as shown in Fig. 3(F), the bottle 2 is rinsed with air. This air-rinsing process is performed by blowing an aseptic air K into the bottle 2 through a nozzle 45, and by the flow of the aseptic air K, foreign material and hydrogen peroxide remaining in the bottle 2 is removed. At that time, the bottle 2 is maintained in a standing attitude with its opening being directed upward. However, as occasion demands, an inverted attitude may be adopted.

As mentioned above, since the bottle is preliminarily sterilized in the shape of the preform and then is mainly sterilized with the hydrogen peroxide, an amount of the hydrogen peroxide to be actually used can be reduced. Accordingly, the rinsing process with hot water, after the air-rinsing process, for washing out the hydrogen peroxide adhering to the bottle is not needed. However, it involves no problem to perform an aseptic water rinsing.

The mist M or gas G of hydrogen peroxide used in the main (final) sterilization is as follows.

### 1) Case: Use of Hydrogen Peroxide Mist M

In order to sterilize a bottle by a conventional technology in which only a main sterilization is performed, it was necessary to adhere the hydrogen peroxide of an amount of 50 *µ*L/500mL bottle to 100 *µ*L/500mL bottle to the bottle. On the contrary, in the case when the preliminary sterilization is performed as in the present invention, a preferred sterilizing effect of 6 Log can be achieved only by adhering the hydrogen peroxide mist M of an amount of 30 *µ*L/500mL to 50 *µ*L/500mL bottle.

### 2) Case: Use of Hydrogen Peroxide Gas G

In order to sterilize a bottle by a conventional technology in which only a main sterilization is performed, it was necessary to blow the hydrogen peroxide gas G having gas density of an amount of 5mg/L to 10mg/L to the bottle. On the contrary, in the case when the preliminary sterilization is performed as in the present invention, a preferred sterilizing effect of 6 Log can be achieved only by blowing the hydrogen peroxide gas G of gas density of 1mg/L to 5mg/L.

After performing the air-rinsing process, as shown in Fig. 3(G), the bottle 2 is filled up with the beverage a from the filling nozzle 10, and as shown in Fig. 3(H), the bottle is sealed with a cap as a lid.

Then, in the manner described above, the bottles 2 formed as packages are collected and shipped to the market.

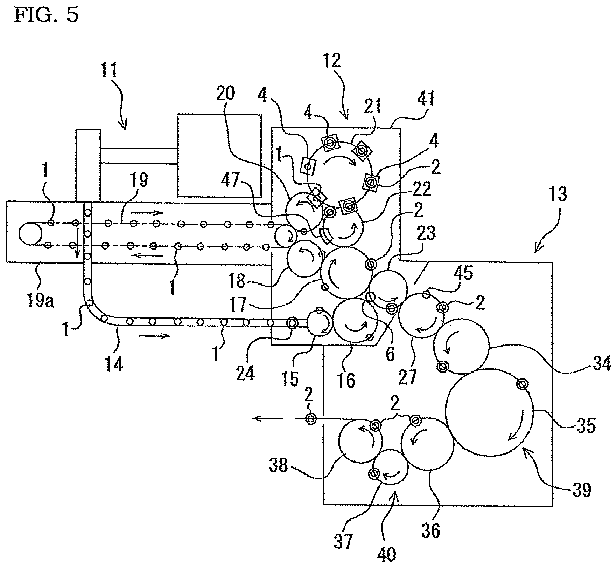

A filling system performing the above filling method has such a structure, for example, as shown in Fig. 5.

As shown in Fig. 5, this filling system is provided with a preform supplying machine 11 for subsequently supplying preforms 1 (Fig. 2(A)), each having a bottomed cylindrical shape having a mouth portion 2a, a blow molding machine 12, and a filling machine 13 for filling the molded bottle 2 with the beverage a, and the bottle 2 is then sealed.

Between the preform supplying machine 11 and the filling machine 13, there are arranged a preform conveying unit for conveying (delivering) the preform 1 on a first conveying passage, a die conveying unit for conveying a die 4 (Fig. 2(C) having a cavity C of a shape conforming with a shape of a product bottle 2 on a second conveying passage connected to the first conveying passage, and a bottle conveying unit for conveying the bottle 2 as a product molded by the die 4 on a third conveying passage connected to the second conveying passage.

The first conveying passage as the preform conveying unit, the second conveying passage as the die conveying unit and the third conveying passage as the bottle conveying unit are linked to each other, and gripper and other members, not shown, for conveying the preforms 1 and the bottles 2 while holding them are disposed on or along these conveying passages.

The preform conveying unit is equipped with preform conveyer 14 for subsequently feeding the preforms 1 at a predetermined interval on the first conveying passage. The preform conveying unit is also equipped with a train of wheels 15, 16, 17 which receive and then transfer the preforms from the terminal end of the preform conveyer 14 and a conveyer 19 which receives the preforms 1 from the wheel 18 and then conveys them.

A nozzle 24 spraying water steam W is disposed on the slightly upstream side from a portion to which the preform conveyer 14 of the preform supplying machine 11 is connected to the wheel 15. The water steam W is jetted from the nozzle 24 toward the preform 1 before being heated (refer to Fig. 2(A)). According to such operation, a thin water film is uniformly formed on the surface of the preform 1. As such nozzle 24, there may be utilized a twin-fluid nozzle which can spray water by using compressed air. According to the use of such nozzle 24, a water film having thickness of preferably 0.02mg/cm² to 1.15mg/cm², and more preferably, 0.07mg/cm² to 0.46mg/cm² is adhered and formed on the surface of each of the preforms 1.

The nozzle 24 may be changed in location or additionally disposed to a predetermined position on the circumferential portion of the wheel 19, for example, before the preform 1 reaches the conveyer 19.

The conveyer 19 includes an endless conveying chain lengthwise extending in the horizontal direction of the conveyer 19, and a heating section 19a is provided along the endless conveying chain. A number of spindles (or mandrels) 43, shown in Fig. 2(B), are mounted to the endless conveying chain at a constant interval, and each spindle (or mandrel) 43 may be rotated while transferring with the endless conveying chain. The spindle (or mandrel) 43 is inserted into the preform 1, which is fed from the wheel 18 side to the conveyer 19, through the mouth portion 2a of the preform 1, and the spindle (or mandrel) 43 is maintained in its standing attitude as shown in Fig. 2(B).

The preform 1 is received by the conveyer 19 through the train of the wheels 15, 16, 17 and 18, and then reciprocates in the heating section 19a by the conveyer 19. On the inner wall surface of the heating section 19a, there is extended a heater 19b (Fig. 2(B)) so as to heat the preform 1 conveyed by the conveyer 19. The preform 1 is rotated together with the rotation of the spindle (or mandrel) which is now transferring on the conveyer 19, and during this operation, the preform 1 can be evenly heated by the heater 19b.

When the preform 1 is heated in the manner mentioned above, the water film adhering on the surface of the preform 1 is also heated by the heating of the heater 19b. Water amount of this water film is, as mentioned before, preferably 0.02mg/cm² to 1.15mg/cm², and more preferably, 0.07mg/cm² to 0.46mg/cm², and according to this heating, the heat-and-humidity sterilization can be performed, so that almost all the bacteria, except sporeforming bacteria having high heat-resisting property, adhering to the surface of the preform 1 are sterilized.

That is, all the portions of the preform 1, except the mouth portion, are heated to a temperature of 90 to 120 °C during about 15 to 30 seconds by the heating of the heating section 19a. On the other hand, it is generally known that almost all the bacteria except the sporeforming bacteria is destroyed and sterilized by about 10-minute heating at 80°C. When this is calculated with Z-value = 5°C, substantially the same sterilizing effect is obtainable as a case in which the heating is performed for 6 seconds at 90°C. Accordingly, when the preform 1 is heated by the heating section 19a in the manner mentioned above, it will be found that almost all the bacterial except the sporeforming bacteria adhering to the surface of the preform 1 can be destroyed and sterilized.

The blow molding machine 12 is equipped with plural sets of dies 4 and blow nozzles 5 (Fig. 2(C) for receiving the preform 1 heated by the heating section 19a of the preform supplying machine 11, and then, heating and molding the preform 1 to the bottle 2.

Within the blow molding machine 12, there is disposed the second conveying passage of the die conveying unit. This second conveying passage is composed of a train of wheels 20, 21, 22, 17 and 23, and the wheel 17 is commonly used between the train of the wheels 20, 21, 22, 17, 23 and another train of the wheels 15, 16, 17, 18.

The plural sets of the dies 4 and the nozzles 5 are located around the wheel 21 and are swiveled around the wheel 21 at a constant speed together with the rotation of the wheel 21.

When the gripper, not shown, of the wheel 20 receives the preform 1 heated by the heating section 19a of the preform supplying machine 11 together with the spindle 43, which are then transferred to the die 4 around the wheel 21, the two split parts of the die 4 are closed and hold the preform 1 as shown in Fig. 2(C). The preform 1 in the die 4 is molded to a final product (bottle) while swiveling together with the die 4 and the blow nozzle 5 around the wheel, by the highly pressurized air-blow for the blow molding process from the blow nozzle 5. Since the preform 1 is uniformly heated to a predetermined temperature by the heater 19b as shown in Fig. 2(B), the blowing process can be smoothly performed.

Furthermore, as mentioned above, since the water amount of the water film adhering to the surface of each of the preforms 1 is preferably 0.02mg/cm² to 1.15mg/cm², and more preferably, 0.07mg/cm² to 0.46mg/cm², the bottle 2 can be formed through the proper blow molding process without being affected with whitening phenomenon, spot generation, wrinkling formation, deformation and the like.

When the preform 1 closely adheres in the cavity C of the die 4 and is formed into a bottle 2, the die 4 is opened at the time when the die 4 contacts the wheel 22, and then, the bottle 2 and the spindle 43 are released. The bottle 2 is then transferred from the spindle 43 to the gripper, not shown, of the wheel 22.

Further, the spindle 43 after releasing the bottle 2 is returned to the conveyer 19 through the wheel 20, and subsequently, holds another preform 1 and then conveys it.

The bottle 2 reaching the wheel 22 from the blow molding machine 12 is subjected to inspection whether the bottle 2 is defective product or not by an inspection device 47 disposed around the peripheral portion of the wheel 22.

The inspection device 47 includes, though not shown, a bottle body portion inspecting unit judging whether the body portion of the bottle 2 is defective or not, a support ring inspecting unit judging whether a support ring 2c (Fig. 1) of the bottle 2 is defective or not, a bottle neck and ceiling portion inspecting unit judging whether the neck and ceiling portion of the bottle 2 is defective or not, a bottle bottom portion inspecting unit judging whether the bottom portion of the bottle 2 is defective or not, and a temperature inspecting unit detecting a temperature of the bottle 2 and judging whether the bottom portion of the bottle 2 is defective or not.

The bottle body portion inspecting unit, the support ring inspecting unit, the bottle neck and ceiling portion inspecting unit and the temperature inspecting unit are arranged circumferentially along the wheel 22.

The bottle body portion inspecting unit, the support ring inspecting unit, and the bottle neck and ceiling portion inspecting unit are provided with lamps and cameras for photographing predetermined portions of the bottle 2 and image processing units for processing the photographed images to thereby discriminate the abnormality of the bottle with respect to the shape, injury, foreign material, color and the like thereof.

The temperature inspecting unit includes a temperature sensor, not shown, to detect a surface temperature of the bottle 2, and in a case when the detected temperature does not reach a predetermined temperature, the bottle is judged as defective product. That is, the bottle 2 of which temperature does not reach the predetermined temperature may be considered not to be subjected to sufficient sterilization even if the sterilization of the hydrogen peroxide be performed thereafter. On the contrary, when the detected temperature of the bottle 2 reaches the predetermined temperature, the bottle 2 can be sufficiently sterilized by the hydrogen peroxide sterilization which will be performed thereafter.

Further, the inspection device 47 is disposed optionally as occasion demands. Furthermore, a bottle body inspection unit, a support ring inspection unit, a bottle neck and ceiling inspection unit and a temperature inspection unit may be selectively arranged as occasion demands.

The bottle 2, which is judged as defective product after the inspection, is rejected from the conveying passage by a rejecting device, now shown, and only acceptable product is conveyed to the wheel 23 from the wheel 22 through the wheel 17.

The sterilization nozzle 6 (see Fig. 3(E)) for performing substantive sterilization is arranged to the circumferential portion of the wheel 23. The sterilization nozzle 6 blows hydrogen peroxide mist M or gas G against the bottle 2 molded by the die 4 to thereby perform the sterilization to the bottle 2 and also sterilize the bacteria or like remaining on the surface of the bottle. In this stage, heat applied to the bottle 2 in the stage of the preform or in the molding stage remains to the bottle 2, and by this remaining heat, the sterilizing effect by the mist M or gas G of the hydrogen peroxide can be enhanced.

As mentioned above, the preliminary sterilization is performed at the stage of the preform 1 shown in Fig. 2(B), and by this preliminary sterilization, almost all the bacteria except some sporeforming bacteria having high heat-resisting property can be sterilized. Accordingly, the bacteria remaining alive in the stage of the preform 1 even by the blowing of the hydrogen peroxide mist M or gas G to the bottle 2 and a few of bacteria which is mixed in the blow molding process and/or conveying process can be subjected to the final sterilizing treatment in the substantive sterilization treatment.

It is to be noted that the sterilization nozzle 6 may be arranged to a predetermined position in the circumference of the wheel 17 or 22 to achieve that the circumferential portion of the wheel 23 is not subjected to corrosion by the sterilizing agent.

Since the preliminary sterilization is performed in the stage of the preform 1 as mentioned above, the amount of the hydrogen peroxide mist M or gas G used in the substantive sterilization stage may be significantly reduced.

The filling machine 13 is provided therein with a third conveying passage of the bottle conveying unit. This third conveying passage is equipped with a train of wheels 27, 34, 35, 36, 37 and 38.

An air rinsing nozzle 45 (see Fig. 3(F)) is disposed on the outer peripheral portion of the wheel 27, and aseptic air K is blown into the bottle through this nozzle 45 to remove the foreign materials and the hydrogen peroxide remaining inside the bottle 2.

Further, a filler 39 for filling the bottle 2 in the aseptic state with the beverage a, and a capper 40 for applying a cap 3 (see Fig. 1) to the bottle 2 filled up with the beverage a is located around the wheel 37 to thereby seal the bottle 2.

Since such filler 39 and capper 40 have structures substantially the same as structures of known ones, the detailed explanation thereof is omitted herein.

Furthermore, a filling system is surrounded by a chamber 41, which is sectioned into an aseptic zone and a gray zone. The preform supply unit 11 and the blow molding machine 12 are disposed in the gray zone, and the filling machine 13 is disposed in the aseptic zone, respectively.

Aseptic air sterilized in a HEPA is always blown into the gray zone, thereby conveying the bottle sterilized in the molding process to the aseptic zone without being secondarily contaminated by bacteria or like.

Next, with reference to Figs. 2, 3 and 5, the operation and function of the filling machine will be explained.

First, the preform 1 is conveyed into the heating section 19a by the operation of the preform conveyer 14 and a wheel train including the wheels 15, 16, 17 and 18.

Before entering the heating section 19a, water steam W is sprayed against the preform 1, as shown in Fig. 2(A), from the nozzle 24 shown in Fig. 5, thus forming a thin water film on the surface of the preform 1. The water amount of the water film formed to each preform 1 is preferably 0.02mg/cm² to 1.15mg/cm², and more preferably, 0.07mg/cm² to 0.46mg/cm².

In the heating section 19a, the preform 1 is uniformly heated, while being conveyed by the conveyer 19, so that the entire temperature of the preform reaches a preferred temperature suitable for the molding process.

Furthermore, the preform 1 with the adhered water film is preliminarily sterilized through the heating in the heating section 19a, and almost all the bacteria, except the sporeforming bacteria, adhering on the surface of the preform 1 can be sterilized.

The preform 1 heated in the heating section 19a and preliminarily sterilized is held by the die 4, as shown in Fig. 2(C), during the passing around the outer periphery of the wheel 21, and swelled so as to have a shape of a product as the bottle 2 in the cavity C by blowing highly pressurized air from the blowing nozzle 5.

After opening the die 4, the molded bottle 2 is taken out therefrom by a gripper provided for the wheel 22, and is inspected whether the molded bottle 2 is defective or not by the inspection unit. Thereafter, the hydrogen peroxide mist M or gas G is blown to the bottle 2 through the sterilization nozzle 6 as shown in Fig. 3(E) at the time of passing around the periphery of the wheel 23, thus being subjected to the substantive sterilizing process.

Since the heat applied in the heating section 19a remains on the bottle 2, the bottle 2 can be effectively sterilized by the hydrogen peroxide mist M or gas G blown through the sterilization nozzle 6. According to this sterilization process, the bacteria remaining on the surface of the bottle can be sterilized.

The thus molded and sterilized bottle 2 is conveyed from the wheel 23 to the wheel 27 disposed on the downstream side, and subjected to the air rinsing treatment by blowing aseptic air K through the nozzle 45 around the wheel 27 as shown in Fig. 3(F).

Thereafter, the bottle 2 is transferred subsequently to a train of wheels 34, 35, 36, 37 and 38 while transferring in the filling machine 13.

The bottle 2 in the filling machine 13 is filled up with the beverage a subjected to the sterilization treatment from the filling nozzle 10 of the filler 39. The bottle 2 filed up with the beverage a is applied with the cap 3 by the capper 40 to be sealed (see Fig. 1 and Fig. 3(H)) and is then discharged through an outlet formed to the chamber 41.

As mentioned above, since the filler 39 and the capper 40 are known ones, the explanation of the beverage filling process and bottle sealing process are omitted herein.

### <Second Embodiment 2>

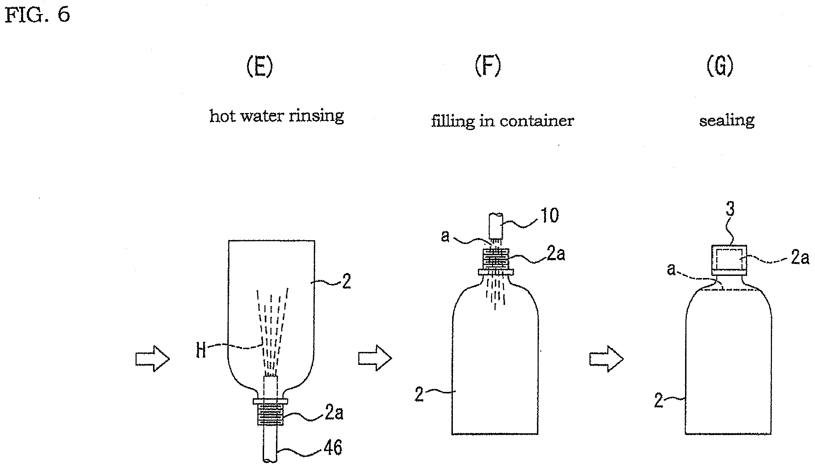

As shown in Fig. 6(E), in this second embodiment 2, the bottle 2 taken out from the die 4 as shown in Fig. 2(D) is rinsed by sterilized hot water H to thereby perform the substantive sterilization. Further, in Fig. 6 (E) , reference numeral 46 denotes a hot water rinsing nozzle for blowing the aseptic hot water H. The temperature of the hot water H is maintained in a range at which the bottle 2 is not deformed.

As in the case of the first embodiment 1, after blowing of the steam to the preform 1 in the stage shown in Fig. 2(B), the preliminary sterilizing is performed by the hear-and-humidity state to thereby sterilizing almost all the bacteria except the sporeforming bacteria already. Accordingly, the bacteria remaining alive in the bottle molding process, but not the sporeforming bacteria, can be sterilized by the hot water rinsing process.

The bottle 2 after the rinsing process is filled up with the beverage a as shown in Fig. 6(F) and then the cap 3 is applied as shown in Fig. 6(G).

The filling method of this second embodiment 2 will be preferred for the filling of beverage, which is not required to be subjected to the sterilization to the sporeforming bacteria, such as acidic drink other than low-acidic drink, carbonated drink, mineral water and the like drink or beverage.

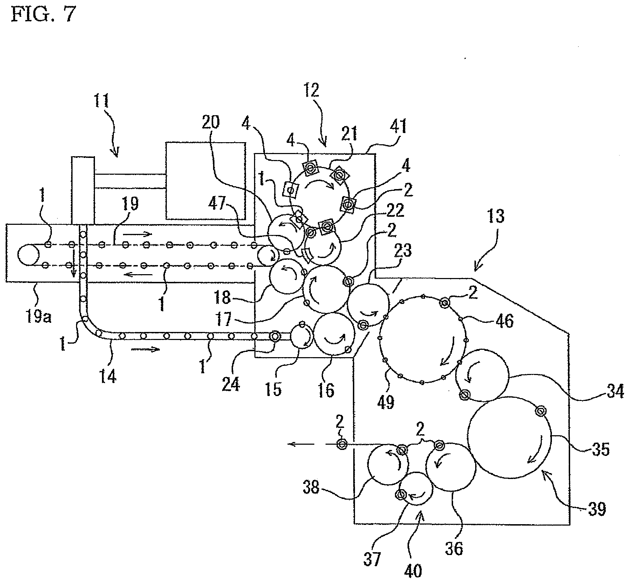

As shown in Fig. 7, the filling system for carrying out the filling method of this second embodiment 2 has a structure in which the wheel 27 in the filling machine 13 of the filling system of the first embodiment 1 shown in Fig. 5 is substituted with a wheel 49.

Around the wheel 29, there are arranged nozzles 46 discharging hot water shown in Fig. 6(E) and grippers, not shown, at predetermined interval from each other, respectively. The gripper of the wheel 49 is rotated vertically in an upside-down motion, and the gripper runs with the bottle 2, shown in Fig. 2(D), being held in a vertically inverted state as shown in Fig. 6(E). In synchronous with this running of the gripper and the bottle 2, the nozzle 46, while running, is inserted into the bottle 2 through the mouth portion 2a to thereby drain the hot water H into the bottle 2. Then, the hot water H fills the inside of the bottle 2, and after the sterilization of the inner surface of the bottle 2, the hot water H flows out of the bottle 2 through the mouth portion 2a.

After the hot water rinsing process, the bottle 2 is filled up with the beverage a through the filling nozzle 10 of the filler 39 while running around the wheel 35 (Fig. 6(F)). The bottle 2 filled up with the beverage a is applied with the cap 3 by the capper 40 to thereby seal the bottle 2 (Fig. 6(G)), which is thereafter discharged outside the filling system.

In this second embodiment 2, like reference numerals are added to portions and members corresponding to those of the first embodiment 1, and detailed explanation thereof is omitted herein.

### <Third Embodiment 3>

A package such as shown in Fig. 1 as a final product can be manufactured by an inline system of this third embodiment 3.

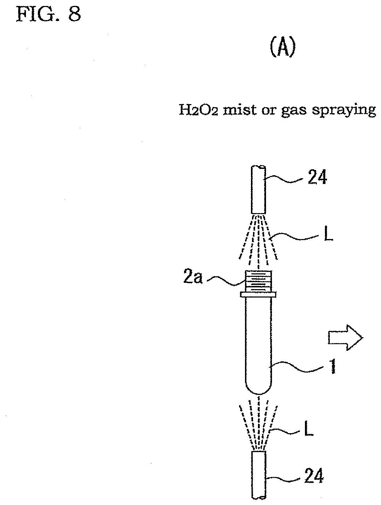

The bottle 2 is formed as a container by substantially the same processes described with reference to the first embodiment, and then, is filled up with a beverage and sealed, thus producing a package. However, in the first embodiment 1, the steam W is sprayed against the preform 1 through the nozzle 24 as shown in Fig. 2(A), whereas in this third embodiment 3, hydrogen peroxide mist or gas L is sprayed against the preform 1 through the nozzle 24 as shown in Fig. 8(A).

The hydrogen peroxide mist or gas L sprayed from the nozzle 24 is produced by a mist generator having a structure like the mist generator 7 shown in Fig. 4.

According to the spraying of the hydrogen peroxide mist or gas L, a condensed hydrogen peroxide film of 35 weight % (reduced quantity) adheres on the surface of the preform 1 in a range of 0.0035 *µ*L/cm² to 0.35 *µ*L/cm², thus forming the film. In a case where the adhering amount (quantity) is less than 0.0035*µ*L/cm², sufficient sterilizing effect cannot be expected even after the heating shown in Fig. 2(B). On the other hand, in a case where this adhering amount is more than 0.35 *µ*L/cm², the bottle 2 may likely be suffered from whitening phenomenon, spot generation, wrinkling formation, deformation and the like defects when the blow-molding is performed thereafter such as shown in Fig. 2(C).

The adhering amount of the condensed film of the hydrogen peroxide of the 35 weight % (reduced quantity) on the surface of the preform 1 is preferably 0.007 *µ*L/cm² to 0.07 *µ*L/cm². The preform 1 with the adhesion of the condensed film of the hydrogen peroxide is conveyed to a section so as be subjected to the heating process as shown in Fig. 2(B).

According to the heating process, the surface of the preform 1 is preliminarily sterilized. That is, under the processes of heating and drying the preform 1 with the adhesion of the hydrogen peroxide condensed film, the density of the hydrogen peroxide adhering to the surface of the bacteria is increased, thus achieving high sterilizing effect, and hence, mold, sporeforming bacteria and the like having high heat-resisting property can be easily sterilized. According to such processes, general bacteria, sporeforming bacteria, fungus such as mold and yeast adhering to the surface of the preform, except those adhering to the mouth portion 2a of the preform 1, can be desirably sterilized.

The preform 1 heated suitably for the subsequent blow-molding process and preliminarily sterilized is then subjected to the blow-molding process as shown in Fig. 2(C), and then, as shown in Fig. 2(D), the bottle 2 as a final product is taken out from the die 4. Thereafter, the bottle 2 is mainly sterilized as shown in Fig. 3(E), and then is subjected to the air-rinsing process as shown in Fig. 3(F). After the air-rinsing process, the bottle 2 is filled up with the beverage a as shown in Fig. 3(G) and the cap 3, as a lid, is applied to the mouth portion 2a of the bottle 2 to thereby seal the interior of the bottle 2 as shown in Fig. 3(H).

The bottles 2 produced as a package through the processes mentioned above are collected and shipped to the market.

Although a filling system for carrying out the filling method mentioned above has a structure like that shown in Fig. 5, the hydrogen peroxide mist or gas L, in place of the water steam, is sprayed against the preform through the nozzle 24. The nozzle 24 is connected to the mist generator having a structure similar to that of the mist generator 7 shown in Fig. 4. According to this structure, the thin condensed film of the hydrogen peroxide is uniformly formed on the surface of the preform 1 (see Fig. 8(A)). The adhering amount of the hydrogen peroxide is preferably 0.0035 *µ*L/cm² to 0.35 *µ*L/cm², more preferably, 0.007 *µ*L/cm² to 0.07 *µ* L/cm² in the case of using the hydrogen peroxide of the 35 weight % (reduced quantity).

The preform 1 is received by the conveyer 19 through the preform conveyer 14 and the train of wheels 15, 16, 17 and 18, and is reciprocally moved within the heating section 19a by this conveyer 19. The heater 19b (see Fig. 2(B)) is arranged in a manner spread on the inner wall surface of the heating section 19a, and the preform conveyed by the conveyer 19 is heated by this heater 19b. The preform 1 is rotated together with the rotation of the spindle (or mandrel) during the transferring on the conveyer 19 to be thereby uniformly heated by the heater 19b.

According to this heating by the heater 19b, the hydrogen peroxide film adhering to the surface of the preform is heated. The adhering amount or quality of the hydrogen peroxide of 35 weight % (reduced quality) as the film is preferably, as mentioned hereinbefore, 0.0035 *µ* L/cm² to 0.35 *µ*L/cm², and more preferably, 0.007 *µ*L/cm² to 0.07 *µ* L/cm², and according to this heating, the density of the hydrogen peroxide adhering to the bacteria surface increases, and thus, almost all the bacteria adhering to the surface of the preform 1, except that adhering to the mouth portion 2a thereof, can be sterilized.

That is, the temperature of the preform 1 heated by the heating section 19b is increased to a temperature of 90 to 120 °C for 15 to 30 seconds except the mouth portion 2a. When the preform 1 is treated by this heating for such time with the adhesion of the hydrogen peroxide of the amount of 0.07 *µ*L/cm², the sterilization effect of 3Log reduction with respect to Basillus subtilis spores as sporeforming bacteria. In addition, to the Chaetomium globsum spores as molds having heat resisting property, the sterilization effect of 6Log reduction can be obtained. Therefore, if the preform 1 is heated at the heating section 19a by the manner mentioned above, almost all the bacteria adhering to the surface of the preform 1, except the mouth portion 2a thereof, can be substantially sterilized.

As mentioned above, the adhering amount or quality of the hydrogen peroxide of 35 weight % (reduced quality) as the film is preferably 0.0035 *µ*L/cm² to 0.35 *µ*L/cm², and more preferably, 0.007 *µ* L/cm² to 0.07 *µ*L/cm², and according to this heating, the density of the hydrogen peroxide adhering to the bacteria surface increases, and thus, almost all the bacteria adhering to the surface of the preform 1, except bacteria or like adhering to the mouth portion 2a thereof, can be sterilized. Thus, at the time of the blow-molding process by the blow molding machine 12, the bottle 2 is free from whitening phenomenon, spot generation, deformation and the like.

Furthermore, as mentioned above, the preliminarily sterilization is performed at the stage of the preform 1 as shown in Fig. 2(B), and through this preliminary sterilization process, almost all the bacteria except that adhering to the mouth portion 2a thereof, can be sterilized. Accordingly, the bacteria remaining alive in the state of the preform 1 and a little amount of bacteria mixing in the blow-molding process and the conveying process can be substantially sterilized by the main sterilizing process through the blowing of the hydrogen peroxide mist M or gas G to the bottle 2.

At the time of this sterilization by the hydrogen peroxide mist M or gas G, since the preliminary sterilization is performed in the preform stage, the used amount of hydrogen peroxide can be effectively reduced. Further, in this third embodiment 3, the main sterilization may be performed by a hot water rinsing process using aseptic hot water H to sterilize the bottle 2 (Fig. 2(D)) taken out from the die 4 as shown in Fig. 6(E).

The present invention is not limited to the embodiments described above and may be carried out by many other various embodiments or modes. For example, a container to which the present invention is applied is not limited to a PET bottle, and many other containers made of resin may be usable. Moreover, the molding process may be performed without being limited to the injection blow molding, and various other blow-molding processes such as direct blow process may be adopted. Furthermore, the conveying means for conveying the preform and the container is not limited to the illustrated wheel conveying train, and various conveying means capable of sequentially conveying the formed containers at a predetermined speed, for example, belt conveyer, bucket conveyer, pneumatic (air) conveyer and the like may be also utilized.

### Reference Numerals

- 1 ---: preform
- 2 ---: bottle
- 3 ---: cap
- 4 ---: die
- 6 ---: sterilization nozzle
- 19b: --- heater
- 21 ---: wheel
- 24 ---: nozzle
- 39 ---: filler
- 40 ---: capper
- a ---: beverage
- G ---: hydrogen peroxide gas
- H ---: aseptic (sterilized) hot water
- M ---: condensed mist of hydrogen peroxide
- W ---: water
- L ---: hydrogen peroxide mist or gas

## Claims

1. A beverage filling method comprising the steps of:
forming a water film on a surface of a preform (1) by spraying water steam (W) while continuously transferring the preform (1), wherein the water film is adhered to each of the preforms (1) of an amount within 0.02mg/cm² to 1.15mg/cm²;
or forming a condensed film of hydrogen peroxide on a surface of the preform (1) by spraying hydrogen peroxide mist or gas (L) while continuously transferring the preform (1),
wherein a condensed film of the hydrogen peroxide of 35 weight % (reduced quantity) is adhered to each of the preforms (1) of an amount within 0.0035µL/cm² to 0.3µL/cm²;
preliminarily sterilizing the preform (1) through a heating and drying process under a state in which the water film or condensed film of the hydrogen peroxide is adhered to the surface of the preform (1);
producing a container (2) through a blow-molding process within a blow-molding die (4) which is continuously being transferred ;
taking out the container (2) from the blow-molding die (4), while continuously transferring the container, a main sterilization is performed ; and
filling, thereafter, the container (2) with a beverage (a) and sealing the container (2) with a lid (3).

2. The beverage filling method according to claim 1, wherein the main sterilization process is performed by blowing condensed mist (M) or gas (G) of hydrogen peroxide to the container (2) to which the heat from the heating of the preform (1) remains, and subsequently, an air rinsing process is performed by rinsing aseptic air (K).

3. The beverage filling method according to claim 1, wherein the main sterilization is performed by a hot water rinsing process using sterilized hot water (H).

4. A beverage filling system, in which there is provided a conveying path for continuously transferring a preform (1) and a container during the processes in which :
a preform (1) is formed to a container (2), a beverage (a) fills the container (2), the container (2) is sealed by a lid (3),
wherein a nozzle (24) for blowing water steam (W) to the preform (1) is foreseen,
wherein a water film is adhered to each of the preforms (1) of an amount within 0.02mg/cm² to 1.15mg/cm² by blowing water steam (W) to the preform;
or wherein a nozzle (24) for blowing hydrogen peroxide mist or gas (L) to the preform (1) is foreseen, wherein a condensed film of hydrogen peroxide of 35 weight % (reduced quantity) is adhered to each of the preforms (1) of an amount within 0.0035µL/cm² to 0.35 µL/cm² by blowing hydrogen peroxide mist or gas (L) to the preform ; the system further comprising, arranged along the conveying path:
a heater (19b) that heats the preform (1) blown with the water steam (W) or hydrogen peroxide mist or gas (L) to thereby preliminarily sterilize the preform (1) at a temperature suitable for a blow-molding process; a molding die (4) for blow-molding the heated preform (1) to the container (2); a main sterilizing unit for sterilizing the blow-molded container (2); a filler (39) for filling the sterilized container (2) with beverage (a); and a capper (40) for sealing the container (2) filled up with the beverage (a).

5. The beverage filling system according to claim 4, wherein the main sterilizing unit includes a sterilizing nozzle (6) for blowing condensed mist (M) or gas (G) of hydrogen peroxide to the container (2) to which the heat from heating the preform (1) remains, and an air rinsing nozzle (45) for performing an air-rinsing process by blowing aseptic air (K) to the container (2) to which the hydrogen peroxide condensed mist (M) or gas (G) was sprayed.

6. The beverage filling system according to claim 4, wherein the main sterilizing unit includes a hot water rinsing nozzle (46) for performing a hot water rinsing process by spraying aseptic hot water (H) to the container (2) to which the heat used for heating the preform (1) remains.

## Patentansprüche

1. Ein Getränkefüllverfahren, aufweisend die Schritte:
Bilden eines Wasserfilms an einer Oberfläche eines Vorformlings (1) durch Sprühen von Wasserdampf (W), während eines kontinuierlichen Transferierens/Bewegens des Vorformlings (1), wobei der Wasserfilm an jedem der Vorformlinge (1) in einer Menge von 0,02 mg/cm² bis 1,15 mg/cm² anhaftet, oder Bilden eines kondensierten Wasserstoffperoxid-Films an einer Oberfläche des Vorformlings (1) durch Sprühen von Wasserstoffperoxid-Nebel oder -Gas (L), während eines kontinuierlichen Bewegens des Vorformlings (1), wobei ein kondensierter Film des Wasserstoffperoxids mit 35 Gewichts-% (reduzierte Menge) an jedem der Vorformlinge (1) in einer Menge von 0,0035 µL/cm² bis 0,35 µL/cm² anhaftet,
vorläufiges Sterilisieren des Vorformlings (1) durch ein Erhitzen-und-Trocknen-Verfahren in einem Zustand, in dem der Wasserfilm oder der kondensierte Wasserstoffperoxid-Film an der Oberfläche des Vorformlings (1) anhaftet,
Herstellen eines Behälters (2) durch ein Blasformverfahren in einem Blasformwerkzeug (4), das kontinuierlich bewegt wird,
Entfernen des Behälters (2) aus dem Blasformwerkzeug (4), wobei während eines kontinuierlichen Bewegens des Behälters eine Hauptsterilisierung ausgeführt wird, und
danach Füllen des Behälters (2) mit einem Getränk (a) und Verschließen des Behälters (2) mit einem Deckel (3).

2. Das Getränkefüllverfahren gemäß Anspruch 1, wobei das Hauptsterilisierungsverfahren durch Blasen von kondensiertem Wasserstoffperoxid-Nebel (M) oder Wasserstoffperoxid-Gas (G) an den Behälter (2) ausgeführt wird, an dem die Wärme von dem Erhitzen des Vorformlings (1) verbleibt, und anschließend ein Luftspülverfahren durch Spülen mit aseptischer Luft (K) ausgeführt wird.

3. Das Getränkefüllverfahren gemäß Anspruch 1, wobei die Hauptsterilisierung durch ein Heißwasserspülverfahren unter Verwendung von sterilisiertem heißem Wasser (H) ausgeführt wird.

4. Ein Getränkefüllsystem, in dem ein Transportweg zum kontinuierlichen Transferieren/Bewegen eines Vorformlings (1) und eines Behälters (2) während der Prozesse bereitgestellt ist, in welchem:
ein Vorformling (1) zu einem Behälter (2) geformt wird, ein Getränk (a) in den Behälter (2) gefüllt wird, der Behälter (2) mit einem Deckel (3) verschlossen wird,
wobei eine Düse (24) zum Blasen von Wasserdampf (W) an den Vorformling (1) vorgesehen ist, wobei ein Wasserfilm an jedem der Vorformlinge (1) in einer Menge von 0,02 mg/cm² bis 1,15 mg/cm² durch Blasen von Wasserdampf (W) an den Vorformling (1) anhaftet, oder wobei eine Düse (24) zum Blasen von Wasserstoffperoxid-Nebel oder -Gas (L) an den Vorformling (1) vorgehsehen ist, wobei ein kondensierter Film von Wasserstoffperoxid mit 35 Gewichts-% (reduzierte Menge) an jedem der Vorformlinge (1) in einer Menge von 0,0035 µL/cm² bis 0,35 µL/cm² durch Blasen von Wasserstoffperoxid-Nebel oder-Gas (L) an den Vorformling (1) anhaftet, wobei das System entlang des Transportweges angeordnet ferner aufweist: einen Heizer (19b), der den Vorformling (1) erhitzt, der mit dem Wasserdampf (W) oder Wasserstoffperoxid-Nebel oder -Gas (L) angeblasen ist/wird, um dadurch den Vorformling (1) bei einer Temperatur, die für ein Blasformverfahren geeignet ist, vorläufig zu sterilisieren, ein Formwerkzeug (4) zum Blasformen des erhitzen Vorformlings (1) in den Behälter, eine Hauptsterilisierungseinheit zum Sterilisieren des blasgeformten Behälters (2), eine Füllvorrichtung (39) zum Füllen des sterilisierten Behälters (2) mit Getränk (a), und eine Verschließvorrichtung (40) zum Verschließen des Behälters (2), der mit dem Getränk (a) gefüllt ist.

5. Das Getränkefüllsystem gemäß Anspruch 4, wobei die Hauptsterilisierungseinheit aufweist eine Sterilisierdüse (6) zum Blasen von kondensiertem Wasserstoffperoxid-Nebel (M) oder Wasserstoffperoxid-Gas (G) an den Behälter (2), an dem die Wärme vom Erhitzen des Vorformlings (1) verbleibt, und eine Luftspüldüse (45) zum Ausführen eines Luftspülverfahrens durch Blasen von aseptischer Luft (K) an den Behälter (2), an den der kondensierte Wasserstoffperoxid-Nebel (M) oder Wasserstoffperoxid-Gas (G) gesprüht wurde.

6. Das Getränkefüllsystem gemäß Anspruch 4, wobei die Hauptsterilisierungseinheit eine Heißwasserspüldüse (46) zum Ausführen eines Heißwasserspülverfahrens durch Sprühen von aseptischem heißem Wasser (H) an den Behälter (2) aufweist, an dem die Wärme verbleibt, die zum Erhitzen des Vorformlings (1) verwendet wurde.

## Revendications

1. Procédé de remplissage de boisson comprenant les étapes suivantes :
la formation d'une pellicule d'eau sur une surface d'une préforme (1) en pulvérisant de la vapeur d'eau (W) tout en transférant en continu la préforme (1), dans lequel la pellicule d'eau adhère à chacune des préformes (1) dans une quantité comprise entre 0,02 mg/cm² et 1,15 mg/cm² ;
ou la formation d'une pellicule condensée de peroxyde d'hydrogène sur une surface de la préforme (1) en pulvérisant de la brume ou du gaz de peroxyde d'hydrogène (L) tout en transférant la préforme (1) en continu,
dans lequel une pellicule condensée de peroxyde d'hydrogène de 35 % en poids (quantité réduite) adhère à chacune des préformes (1) dans une quantité comprise entre 0,0035 µL/cm² et 0,35 µL/cm² ;
la stérilisation préalable de la préforme (1) par le biais d'un processus de chauffage et de séchage dans un état dans lequel la pellicule d'eau ou la pellicule condensée du peroxyde d'hydrogène adhère à la surface de la préforme (1) ;
la production d'un récipient (2) à l'aide d'un processus de moulage par soufflage à l'intérieur d'une matrice de moulage par soufflage (4) qui est transférée en continu ;
le retrait du récipient (2) de la matrice de moulage par soufflage (4), tout en transférant en continu le récipient, une stérilisation principale est effectuée ; et
le remplissage, par la suite, du récipient (2) avec une boisson (a) et la fermeture hermétique du récipient (2) avec un couvercle (3).

2. Procédé de remplissage de boisson selon la revendication 1, dans lequel le processus de stérilisation principal est effectué en soufflant de la brume (M) ou du gaz (G) condensé(e) de peroxyde d'hydrogène vers le récipient (2) sur lequel subsiste la chaleur du chauffage de la préforme (1), et par la suite, un processus de rinçage à l'air est réalisé à l'aide d'air aseptique de rinçage (K).

3. Procédé de remplissage de boisson selon la revendication 1, dans lequel la stérilisation principale est effectuée à l'aide d'un processus de rinçage à l'eau chaude utilisant de l'eau chaude stérilisée (H).

4. Procédé de remplissage de boisson, dans lequel est prévu un trajet d'acheminement pour transférer en continu une préforme (1) et un récipient au cours des processus dans lesquels :
une préforme (1) est formée sur un récipient (2), une boisson (a) remplit le récipient (2), le récipient (2) est fermé hermétiquement par un couvercle (3),
dans lequel est prévue une buse (24) servant à souffler de la vapeur d'eau (W) vers la préforme (1),
dans lequel une pellicule d'eau adhère à chacune des préformes (1) dans une quantité comprise entre 0,02 mg/cm² et 1,15 mg/cm² en soufflant de la vapeur d'eau (W) vers la préforme ;
ou dans lequel est prévue une buse (24) destinée à souffler de la brume ou du gaz (L) de peroxyde d'hydrogène vers la préforme (1),
dans lequel une pellicule condensée de peroxyde d'hydrogène de 35 % en poids (quantité réduite) adhère à chacune des préformes (1) dans une quantité comprise entre 0,0035 µL/cm² et 0,35 µL/cm² en soufflant de la brume ou du gaz de peroxyde d'hydrogène (L) vers la préforme ; le système comprenant en outre, disposé le long du trajet d'acheminement :
un élément chauffant (19b) qui chauffe la préforme (1) soufflée avec la vapeur d'eau (W) ou la brume ou le gaz de peroxyde d'hydrogène (L) afin de stériliser préalablement la préforme (1) à une température adéquate pour un processus de moulage par soufflage ; une matrice de moulage (4) pour mouler par soufflage la préforme chauffée (1) sur le récipient (2) ; une unité de stérilisation principale pour stériliser le récipient moulé par soufflage (2) ; un élément de remplissage (39) pour remplir le récipient stérilisé (2) avec la boisson (a) ; et une machine à capsuler (40) pour fermer hermétiquement le récipient (2) rempli avec la boisson (a).

5. Système de remplissage de boisson selon la revendication 4, dans lequel l'unité de stérilisation principale comporte une buse de stérilisation (6) pour souffler de la brume (M) ou du gaz (G) condensé(e) de peroxyde d'hydrogène vers le récipient (2) sur lequel subsiste la chaleur provenant du chauffage de la préforme (1), et une buse de rinçage à l'air (45) servant à effectuer un processus de rinçage à l'air en soufflant de l'air aseptique (K) dans le récipient (2) sur lequel de la brume (M) ou du gaz (G) condensé(e) de peroxyde d'hydrogène a été pulvérisé.

6. Système de remplissage de boisson selon la revendication 4, dans lequel l'unité de stérilisation principale comporte une buse de rinçage à l'eau chaude (46) pour effectuer un processus de rinçage à l'eau chaude par pulvérisation d'eau chaude aseptique (H) sur le récipient (2) sur lequel subsiste la chaleur utilisée pour chauffer la préforme (1).
